# EUROPEAN PATENT SPECIFICATION

(11) **EP 2 509 686 B1**
(45) Date of publication and mention of the grant of the patent: **13.08.2014**
(21) Application number: 10784425.0
(22) Date of filing: 24.11.2010
(51) Int. Cl.: A61N 7/00, A61N 7/02, A61B 19/00

(54) **DEVICES FOR ADIPOSE TISSUE REDUCTION AND SKIN CONTOUR IRREGULARITY SMOOTHING**
VORRICHTUNGEN FÜR FETTGEWEGEREDUKTION UND EBNUNG VON UNREGELMÄSSIGEN HAUTKONTUREN
DISPOSITIFS DE RÉDUCTION DU TISSU ADIPEUX, ET LISSAGE DES IRRÉGULARITÉS DU CONTOUR DE LA PEAU

(30) Priority: 10.12.2009 US 634749
(43) Date of publication of application: 17.10.2012
(73) Proprietor: General Electric Company, Schenectady, NY 12345 (US)
(72) Inventor: DAVIS, Cynthia Elizabeth, Landberg, Niskayuna, NY 12309 (US); FAN, Ying, Niskayuna, NY 12309 (US)
(74) Representative: Bedford, Grant Richard
(86) International application number: PCT/US2010/057996
(87) International publication number: WO 2011/071703

(56) References cited:
- WO-A1-03/070105
- WO-A1-2007/093998
- WO-A2-2008/144274
- WO-A2-2009/050719
- US-A1- 2009 227 910

## Description

### BACKGROUND

The invention relates to adipose tissue reduction, and more particularly to methods and systems for adipose tissue reduction and skin contour irregularity smoothing.

Various body contouring systems exist today that attempt to remove or destroy subcutaneous fat tissue (or adipose tissue) from a person's body. Some systems are invasive, such as liposuction, where a device is inserted into the body and the device physically removes adipose tissue through suction. Other systems are non-invasive, for example, ultrasound based systems. Non-invasive systems are preferred due to relative comfort for the patient while receiving the therapy, and also there is usually no need for a recovery time.

In some cases after a body contouring procedure the adipose tissue reduction is often followed by one or more treatments to facilitate skin tightening or contour irregularity smoothing which are carried out as procedures separate from the procedure for reducing adipose tissue. Skin tightening may be achieved by heating connective tissues in an area where adipose tissue reduction is carried out. Typically, adipose tissue reduction and skin tightening are performed using different equipment. For example, adipose tissue reduction may be performed by liposuction, whereas skin tightening may be achieved by radio frequency treatment.

Due to the need for a different system to carry out the two procedures, the patient is often required to visit the physician separately for adipose tissue reduction, and skin tightening. Usually the skin tightening procedure is done at a later date after adipose tissue reduction treatment. In addition, even if an attempt is made to carry out the two treatments during a single visit of the patient to the clinic the patient or the equipment needs to be shifted from one location to another within the clinic, for example, to accommodate the need for a different system/technique to carry out the two different procedures. None of the existing procedures provide a one step solution for a patient.

US 2009/0227910 teaches a similar device operating at 0.5-20 mHz.

Therefore, it would be desirable to provide a single step solution to the multiple step problem of either removing or reducing unwanted tissue volume while simultaneously providing for improved cosmetic appearance by skin contour smoothening.

### BRIEF DESCRIPTION

The invention is defined in claim 1.

In one example, an ultrasound device disposed in a housing is provided. The ultrasound device comprises a first transducer for imaging a region of interest, and a second transducer that generates one or more ultrasound frequencies for cavitating fat cells in the region of interest, and one or more frequencies for thermally treating connective tissues in the region of interest.

In another example, an ultrasound device is provided. The ultrasound device comprises a first transducer for imaging a region of interest, a second transducer that generates one or more ultrasound frequencies for cavitating adipose cells in the region of interest, and a third transducer that generates one or more frequencies for thermally treating connective tissues in the region of interest.

A system for cavitating adipose tissue and treating adipose and connective tissues in a region of interest is also discussed. The system allows a user to simultaneously or sequentially cavitate and treat various tissues in a region of interest. The system comprises a transducer housing, comprising a first transducer for imaging an area in a region of interest to determine parameters pertaining to the adipose tissue and the non-adipose tissues, a second transducer that generates one or more ultrasound frequencies for cavitating adipose tissues in the region of interest, and one or more frequencies for thermally treating adipose and connective tissues in the region of interest, a thermal treatment component for providing a thermal treatment to at least a portion of adipose and connective tissues in the region of interest, and a controller that enables the user to selectively control the first and second transducers and the thermal treatment component.

A non-invasive method for substantially contemporaneously providing an ultrasound based cavitation treatment and an ultrasound based thermal treatment in a region of interest using the invention, comprising imaging at least a portion of the region of interest to collect one or more parameters relating to one or more of, adipose tissue, non-adipose tissue, and skin, cavitating at least a portion of the adipose tissues in the region of interest, and thermally treating at least a portion of the adipose and connective tissue in the region of interest, is also discussed.

### DRAWINGS

These and other features, aspects, and advantages of the invention will become better understood when the following detailed description is read with reference to the accompanying drawings in which like characters represent like parts throughout the drawings, wherein:
FIGS. 1-2 are schematic diagrams of two embodiments of an ultrasound device of the invention for cavitating and thermally treating a region of interest;
FIG. 3 is a block diagram of an ultrasound system of the invention;
FIG. 4 illustrates transducer arrays that may be used to deliver the therapy to the region of interest; and
FIG. 5 is a flow chart for an example of a method of the invention for providing therapy to the region of interest.

### DETAILED DESCRIPTION

The invention addresses issues of patients concern when undergoing adipose tissue reduction. One of the main concerns of a patient deciding to undergo adipose tissue reduction is the resultant loose or contoured skin caused by sudden loss of fat from underneath the skin. In certain embodiments, the method enables the patient to undergo adipose tissue reduction and skin tightening or skin contour smoothening in the same therapy session in a clinic, for example. The fat reduction and skin tightening can be done substantially contemporaneously. In this manner, some of the systems and methods offer solutions that are less complicated and time saving alternatives to existing procedures for adipose tissue reduction and skin contour smoothening or skin tightening. In addition, both adipose tissue reduction and skin contour smoothening can be provided using a single device. Use of a single device for delivering both the treatments makes it convenient for the patient receiving the treatment as well as for the technician or user delivering the treatment. For example, the patient is not required to be shifted from one location to another when the treatment is switched from adipose tissue reduction to skin contour smoothening, or the user does not need to move between the bulky equipment for adipose tissue reduction and skin contour smoothening when changing from one treatment to another. In certain embodiments, the fat cells may be cavitated to reduce the fat, and the connective tissues may be thermally treated for skin tightening.To more clearly and concisely describe the subject matter of the claimed invention, the following definitions are provided for specific terms, which are used in the following description and the appended claims. Throughout the specification, exemplification of specific terms should be considered as non-limiting examples.

As used herein, "cavitating" refers to treating tissue (such as fat tissue) primarily with cavitational mechanism. However, cavitating may also have thermal effects on the tissue. The tissue may be destroyed either by cavitation or thermal effect. HIFU parameters may be adjusted such that the majority of the damage to tissue is cavitational in nature, but there may be thermal effects or damages to the tissue.

As used herein, "thermally treating" refers to treating tissue primarily with a mechanism that is thermal in nature. However, thermally treating may have cavitational effects.

As used herein, "adipose tissue" means subcutaneous, visceral or other tissues made primarily of fat cells. Adipose tissues may also comprise connective tissues, blood vessels and other structures. Adipose tissue may be white adipose tissue or brown adipose tissue.

As used herein, "connective tissues" are found either in the adipose tissue or in the skin surrounding the adipose tissue.

As used herein, "patient" means a person receiving the treatment for adipose tissue reduction and skin contour smoothening.

As used herein, "region of interest" means one or more sites on the patient targeted for receiving the therapy. The region of interest may include, but is not limited to, a subcutaneous region of interest or an inner region or visceral region.

Terms "skin tightening", "skin contour smoothening", "contour smoothening", "contour irregularity smoothing" may be used interchangeably throughout the application, and are non-limiting.

A "therapy" as used herein may comprise a combination of treatments, for example, two different kinds of treatments such as, but not limited to, adipose tissue reduction, and skin contour smoothening, wherein the two procedures may be provided substantially contemporaneously.

As used herein, "substantially contemporaneously" means in a successive manner, without any compulsory time delays between the two treatments of adipose tissue reduction and skin tightening that are otherwise caused due to the requirement of the system or the method for treatment. For example, the therapy does not include any compulsory time delays, which are required when adipose tissue reduction, and skin contour smoothening employ different devices. The compulsory time delays in such cases may be to accommodate shifting of the patient from one device to another when switching from one treatment to another. The time delays may also include a subsequent appointment of the patient with the user, to receive the second treatment (skin contour smoothening) as it may not be feasible in the clinic settings to obtain the two different treatments from two different devices by one or more users on the same day. As used herein, "substantially contemporaneously" may also include simultaneously. However, cavitating and thermally treating may not be carried out in a successive manner. In some instances, the skin tightening (thermally treating) may be performed after a time delay after cavitating the adipose tissue for adipose tissue reduction, to allow the body to remove the treated adipose tissue (such as fat cells). In cases of multiple treatment sessions, the skin tightening may be performed on the second and subsequent treatments to tighten the skin in and around the regions where cavitating was carried out during the previous treatment session.

As used herein, "user" means one or more persons (technologist, nurse or physician) operating the system to provide therapy to the patient.

A "therapy session," as used herein, is a period of time in which a patient receives therapy, e.g. for adipose tissue reduction and skin contour smoothening for a determined portion of the body. The entire therapy session for the determined portion of the body comprises of a single sitting, and the patient or the delivery device is not required to move around to accommodate the need for a different device to deliver the therapy. For example, a therapy session may include one visit by the patient to the user of the system. Therefore, the therapy sessions are time efficient and convenient for the recipient of the therapy, that is, the patient. However, a therapy session may include an extended period of time, where the extended period is required to cover a large treatment space of the body to deliver the therapy. The therapy session may also include any planned/intended time delays, or gaps between the therapy sessions, or between the two treatments, which are not because of the requirement of the system or the method for delivering the therapy.

In certain embodiments, the reduction of adipose tissue may be achieved by destroying the fat cells via apoptotic or necrotic methods utilizing heat or cavitating. In fat cell cavitation, the ultrasound energy causes damage to the fat cells via vibrational mechanism causing the cells to break or lyse. As used herein, the term "lysis" refers to the death of a cell by breaking of the cellular membrane, causing the contents to flow out, often by viral, enzymic or osmotic mechanisms thereby compromising the integrity of the cell. Subsequent thermal treatment of the surrounding connective tissues facilitates skin contour smoothening. Skin contour smoothening is achieved by shrinking the connective tissues with thermal damage that serves to denature collagen fibrils. The connective tissue may be located in the treated adipose tissue, nearby adipose tissue or skin layer. According to some embodiments, the method may further affect surrounding tissue, such as muscle tissue, connective tissue, blood vessels, nerve tissue, fat tissue, adipose tissue and any combinations thereof.

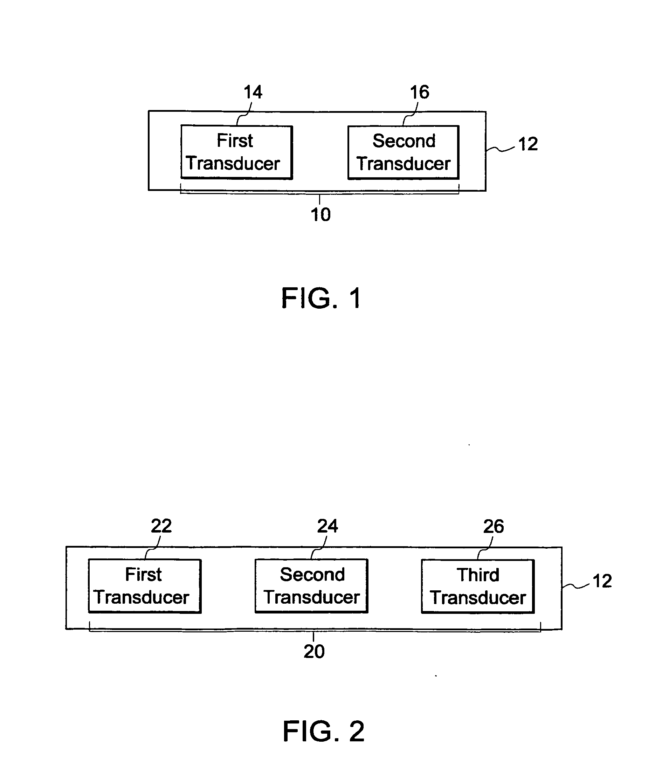

FIG. 1 illustrates an ultrasound device 10 disposed in a housing 12. The ultrasound device 10 provides therapy to a region of interest in a patient. The housing 12 comprises a first transducer 14 for imaging a region of interest. The housing 12 comprises a second transducer 16 for delivering one or more ultrasound frequencies to the region of interest for cavitating at least a portion of adipose cells/tissue in the region of interest, and one or more frequencies for thermally treating at least a portion of connective tissue surrounding the cavitated fat cells in the region of interest. Thermally treating the connective tissues facilitates skin tightening. In addition, mechanical pressure may be applied using the ultrasound probe to help smoothen the skin. In one embodiment, the second transducer generates high intensity focused ultrasound (HIFU). The cavitating frequency is in a range from 100 KHz to 3 MHz, or from about 250 KHz to about 2 MHz. The thermal treating frequency is in a range from about 1 MHz to about 30 MHz, or from about 1 MHz to about 10 MHz.

The second transducer 16 may switch between a pulse mode and a continuous-wave mode. For example, the second transducer 16 may operate in pulse mode to provide pulses of HIFU for cavitating the adipose tissue, and operate in continuous mode to provide continuous wave (CW) for thermally treating the region of interest.

The first transducer 14 operates at least in part as a receiver for the purpose of imaging to receive the acoustic energy transmitted by the second transducer 16. For imaging purposes, for example, the second transducer may emit acoustic energy in a frequency range from about 100 KHz to about 3 MHz. In one example, the back-scattered energy, caused as a result of energy transmitted by the second transducer 16 for thermally treating the connective tissues, may be received by the first transducer for imaging the region of interest.

FIG. 2 illustrates an example of an ultrasound device for providing the therapy. The ultrasound device 20 has separate transducers for cavitating and thermally treating the region of interest. The ultrasound device 20 comprises a first transducer 22 for imaging a region of interest; a second transducer 24 for generating one or more ultrasound frequencies for cavitating adipose cells in the region of interest, and a third transducer 26 for generating one or more frequencies for thermally treating connective tissues in the region of interest. The three transducers 22, 24 and 26 are disposed in a housing 28. In one embodiment, the housing 28 is a probe housing. At least one of the second and third transducers 24 and 26, respectively, generates HIFU. The second transducer 24 operates at a frequency in a range from about 100 KHz to about 3 MHz. The third transducer 26 operates at a frequency in a range from about 1 MHz to about 30 MHz.

In certain embodiments, the first transducer 22 may rely upon the second transducer to transmit acoustic energy, which is then back-scattered and received by the first transducer 22 to produce an image of the region of interest. The third transducer 26 may or may not transmit ultrasound energy. For example, the third transducer 26 may include a laser source, a radio frequency (RF) source, an ultrasound transducer, infrared source, microwave source, or combinations thereof to thermally treat the connective tissues.

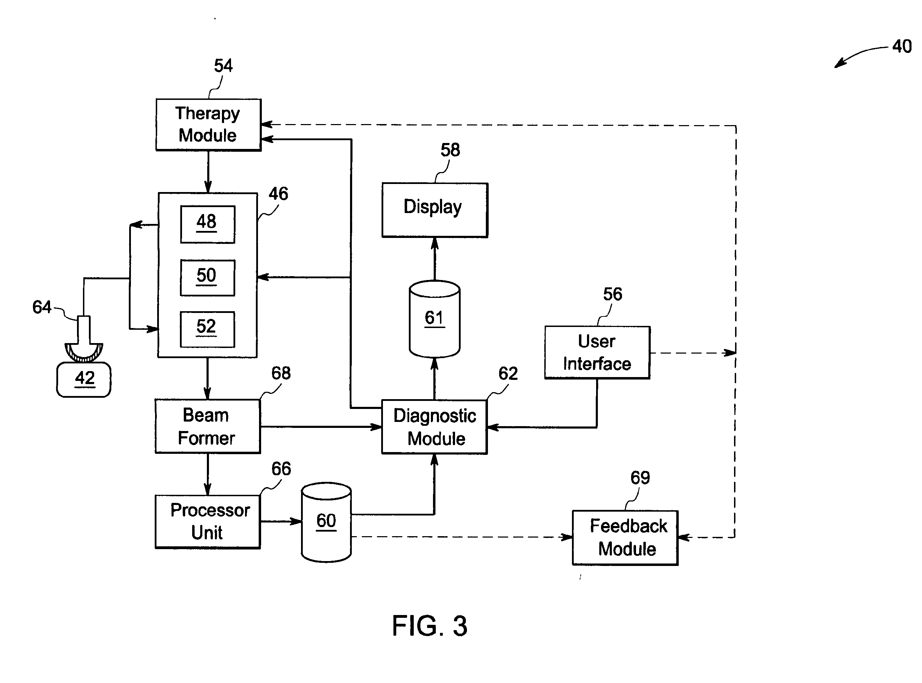

FIG. 3 illustrates an example of an ultrasound system 40 that can display images of the region of interest 42 and provide therapy to the region of interest 42 as described in more detail below. The system 40 allows a user to substantially contemporaneously, simultaneously, or sequentially cavitate and thermally treat various tissues in a region of interest. In one example, the system 40 may be a console-based ultrasound system that may be provided on a movable base.

The system 40 comprises a transducer housing 46 for housing a first transducer 48, a second transducer 50, and a thermal component 52. The first transducer 48 may image the region of interest 42 to determine parameters pertaining to the adipose tissue and the non-adipose tissues. The first transducer 48 may image the region of interest 42 before applying the therapy, or after applying the therapy, or while applying the therapy. The second transducer 50 generates one or more ultrasound frequencies for cavitating adipose cells in the region of interest 42, and one or more frequencies for thermally treating, connective tissues in the region of interest 42. The second transducer 50 may also generate one or more frequencies for imaging the region of interest 42. Although not illustrated, the second transducer 50 may also function to provide thermal treatment, thereby eliminating the need for a separate third component 52. In embodiments where the second transducer may also function as the third component 52, the second transducer 50 may generate one or more frequencies for thermally treating the connective tissues to shrink the connective tissues in the region of interest 42. While only one region of interest 42 is depicted, the first and/or second transducers 48 and 50, respectively, may be configured to operate in a plurality of regions of interest. The thermal treatment component 52 may provide a thermal treatment to at least a portion of connective tissues around the adipose tissues in the region of interest 42.

The first and second transducers 48 and 50 comprise an array of transducer elements that emit ultrasonic signals. The transducer elements can comprise a piezoelectrically active material, such as lead zirconante titanate (PZT), lithium niobate, lead titanate, barium titanate, and/or lead metaniobate, or combinations thereof. Alternatively, the piezoelectrically active component of the transducer element may comprise one or more of a piezoelectric ceramic, a piezoelectric crystal, piezoelectric plastic, and/or piezoelectric composite materials. In addition to, or instead of, a piezoelectrically active material, transducers 48 and 50 can comprise any other materials configured for generating radiation and/or acoustical energy such as capacitively coupled transducers or other acoustic sources. Transducers 48 and 50 can also comprise one or more matching and/or backing layers configured along with the transduction element such as coupled to the piezoelectrically active material. Transducers 48 and 50 can also be configured with single or multiple damping elements along the transducer element(s).

The first transducer 48 receives ultrasonic signals in response to ultrasonic signals transmitted by the second transducer 50 for imaging purposes. In one example, the second transducer 50 may deliver low energy acoustic energy during imaging and high-energy acoustic pulses during therapy. The imaging transducer or the first transducer 48, in this example, operates in a frequency range from about 0.2 MHz to about 30 MHz, or from about I MHz to about 10 MHz. The imaging signals are back-scattered from physiological structures in the body, for example, adipose tissue, muscular tissue, blood cells, veins or objects within the body (e.g., a needle, an implant) to produce echoes that return to the transducer elements. The imaging signals are received by the receiver of the first transducer 48. The received echoes are provided to a beamformer 68 that performs beamforming and outputs an RF signal, for example. The RF signal is then provided to a processor unit 66 that processes the RF signal.

The second transducer 50 may operate in a range of frequencies depending on whether the second transducer 50 is transmitting for cavitating or thermally treating, or both. For example, the second transducer 50 may operate in a frequency range from about 100 KHz to about 3 MHz for cavitating the adipose tissues. In one embodiment where the second transducer 50 is configured to function as a thermal treatment component, that is, where the second transducer 50 is required for both cavitating and thermally treating, the second transducer 50 may operate in a frequency range from about 0.75 MHz to about 1.25 MHz. However, in instances where the second transducer 50 is required for cavitating, and the third component 52 is required for thermally treating, and the third component 50, is an ultrasound transducer, the second transducer 50 may operate in a frequency range from about 100 KHz to about 3 MHz, and the third component 52 may operate in a frequency range from about 1 MHz to about 30 MHz. When the thermal component 52 is not an ultrasound transducer, the thermal component 52 may be one or more of a laser source, a radio frequency (RF) source, an ultrasound transducer, infrared source, microwave source, or combinations thereof.

A therapy module 54 is used to control the delivery of therapy to the treatment locations based on one or more therapy parameters. The therapy module 54 is connected to a user interface 56, such as a mouse, keyboard, and controls operation of the transducer housing 46. The therapy module 54 is configured to receive inputs from a user using the user interface 56. In one embodiment, the therapy module 54 may automatically move the treatment location between multiple points based on user inputs or tracking input from a tracking module which may be based upon optical, infrared, electromagnetic or other tracking technologies. The therapy module 54 of the system 40 may also be used to enable the user to selectively control the first and second transducers and the thermal treatment component 48, 50 and 52, respectively.

The therapy module 54 may be configured to receive imaging commands from the user. The therapy module 54 may receive imaging and/or therapy commands from the user through a user interface 56 for applying therapy to the region of interest 42. The user may provide instructions on whether to image the region of interest 42, or provide therapy to the region of interest 42. Further, the user can specify whether to cavitate or provide thermal treatment for the region of interest 42 or the module will determine the proper treatment parameters based upon the tissue and treatment desired. The delivery of therapy may be based upon therapy commands provided by the user. The user may instruct the system 40 using the user interface 56. The user interface 56 may be a touchscreen, keyboard, or a mouse. For example, the touchscreen may allow the operator or user to select options by touching displayed graphics, icons, and the like.

A therapy command may comprise any factor or value that may be determined by the system 40 or any input that may be entered by the user that affects the therapy applied to the region of interest 42. In some embodiments, the system 40 may automatically differentiate the adipose tissue and the non-adipose tissue (such as connective tissue, skin and muscle). The system 40 may also automatically display to a viewer (such as the user or the patient) a boundary between the adipose tissue and between the adipose tissue and the non-adipose tissue by overlaying the image with a graphical representation that indicates the boundary. Furthermore, the system 40 may automatically display to a viewer of the system 40 the region of interest 42 within the image where therapy may be applied (or is recommended by the system 40 to be applied). In addition, the user may be able to modify the treatment space 40 that is automatically displayed by the system 40 through user inputs.

A therapy command may comprise a transducer parameter that relates to the configuration or operation of the transducer elements (not shown) or probe 64. Examples of the therapy command may comprise parameters of the ultrasound transducers 48, and 50, or time period for applying the therapy. The terms "therapy commands" and "therapy parameters" may be used interchangeably throughout the application, and refer, for example, to the settings of the system or the factors regarding the patient that are taken into account for delivering the therapy. A variety of geometries may be used and the probe 64 may be provided as part of, for example, different types of ultrasound probes. For example, therapy command may include instructing the system 40 to deliver low energy pulses during imaging and high energy pulses during therapy.

Examples of transducer parameters include, but are not limited to, a focal depth of the ultrasound beam, a focal region size, an ablation time for each point within the region of interest that receives therapy, an energy level of the therapy signals, and a rate of focal region movement within the ROI during the therapy session. The transducer parameters may also include a frequency or intensity of the therapy ultrasound signals, power, peak rarefactional pressure, pulse repetition frequency and length, duty cycle, depth of field, waveform used, speed of beam movement, density of beam, cavitation priming pulse, and general pulse sequence parameters. Also, therapy commands may include anatomical parameters, such as the location, shape, thickness, and orientation of adipose tissue and non-adipose tissues. An anatomical parameter may also include a density of the adipose tissue and the non-adipose tissues. Furthermore, therapy parameters include the type of probe 64 used during the therapy session. The age, gender, weight, ethnicity, genetics, or medical history of the patient may also be examples of therapy commands. After therapy has been applied to a region of interest 42, the system 40 or the operator/user may adjust the therapy parameters before applying therapy to the same region of interest 42 again, or to another region of interest.

The therapy module 54 may be implemented utilizing any combination of dedicated hardware boards, DSPs, processors, etc. Alternatively, the therapy module 54 may be implemented utilizing an off-the-shelf PC with a single processor or multiple processors, with the functional operations distributed between the processors. As a further option, the therapy module 54 may comprise a hybrid configuration in which certain modular functions are performed utilizing dedicated hardware, while the remaining modular functions are performed utilizing an off-the-shelf PC and the like.

While the therapy module 54 is configured to deliver a therapy to the treatment locations based on one or more therapy parameters. The diagnostic module 62 is configured to control the probe 64 to obtain diagnostic ultrasound signals from the region of interest 42.

The processor unit 66 processes the acquired ultrasound information (e.g., RF signal data or IQ data pairs) and prepares frames of ultrasound information for display on a display 58. The display 58 may comprise one or more monitors that present patient information, such as diagnostic and therapeutic ultrasound images, to the user for review, diagnosis, analysis, and/or treatment. The display 58 may automatically display, for example, a (two dimensional) 2D, (three dimensional) 3D, or (four dimensional) 4D ultrasound data set stored in the memory 60 or currently being acquired, this stored data set may also be displayed with a graphical representation (e.g., an outline of a treatment space or a marker within the treatment space).

The processing unit 66 may receive ultrasound data in one of several forms. For example, in the embodiment, the received ultrasound data constitutes IQ data pairs representing the real and imaginary components associated with each data sample. The data may be processed by the processing unit 66 by employing one or more of a color-flow module, an acoustic radiation force imaging (ARFI) module, a B-mode module, a spectral Doppler module, an acoustic streaming module, a tissue Doppler module, a C-scan module, and an elastography module. Other modules may be included, such as an M-mode module, power Doppler module, harmonic tissue strain imaging, among others. However, embodiments described herein are not limited to processing IQ data pairs. For example, processing may be done with RF data and/or using other methods. Furthermore, data may be processed through multiple modules.

Each of the modules are configured to process the IQ data pairs in a corresponding manner to generate color-flow data, ARFI data, B-mode data, spectral Doppler data, acoustic streaming data, tissue Doppler data, C-scan data, elastography data, among others, all of which may be stored in a memory 60 temporarily before subsequent processing. As an example, it may be desired to view different ultrasound images relating to a therapy session in real-time on the display 58.

The processing unit 66 is adapted to perform one or more processing operations according to a plurality of selectable ultrasound modalities on the acquired ultrasound information. Acquired ultrasound information may be processed in real-time during a scanning or therapy session as the echo signals are received. Additionally or alternatively, the ultrasound information may be stored temporarily in the memory 60 during a scanning session and processed in less than real-time in a live or off-line operation. The image memory 61 is included for storing processed frames of acquired ultrasound information that are not scheduled to be displayed immediately, for example. The image memory 61 may comprise any known data storage medium, for example, a permanent storage medium, removable storage medium, etc.

After or while providing therapy to an area within the region of interest 42, the user may determine, whether the therapy is complete for the region of interest 42 and if the region of interest should be moved to another point within the patient. Automatic determination of whether the treatment space has been sufficiently treated or completed may be determined by, for example, elasto-graphic methods. Alternatively, the user or a feedback module 69 may determine whether the therapy is complete. If the therapy is complete for a given region of interest, the feedback module 69 may determine the next region of interest where the probe 64 should be moved.

The feedback module 69 may be coupled to the processing unit 66. In addition, the feedback module 69 may also be coupled to one or more of the display 58, memory 60, image memory 61, or the user interface 56. The feedback module 69 may compare the actual output of the system with the desired output. The actual output refers to the result of the therapy delivered to the region of interest. The actual output may be provided as displayed images, or images stored in the memory 60 or 61, or the data related to the displayed or stored images. The desired output may be in a tabular form, or images, that may be stored in the memory 60 or 61 for example. The desired output may be specified by the user. For example, the desired output may be specified by the user depending on the amount of adipose tissue to be reduced.

The feedback module 69 may compare the actual output and the desired output and inform/alert the system if required. In one example, the feedback module 69 may also use the therapy commands provided to the system 40 by the user to determine the acceptable levels of adipose tissue reduction and thermal treatment, and accordingly notify the system when such limits are exceeded or approaching. In one example, the feedback module 69 may alert the system by beeping, to caution the user, for example, if the determined limit of adipose tissue to be treated exceeds or is about to exceed or if an area has been previously treated. In one embodiment, the feedback module 69 may have built in intelligence that may alter the therapy parameters to amend the therapy being provided if required.

The feedback module 69 may take the data in the processing unit 66, displayed images (on the display 58), or stored images (in the memory 61) as the input and make a decision whether or not the data or the images are acceptable. For example, the feedback module 69 may use the displayed images to determine whether the amount of adipose tissue cavitated in the region of interest 42 is sufficient to stop the therapy in the region of interest 42. The feedback module 69 may either provide feedback after completion of the therapy, or during the therapy. In one example, the feedback module 69 may verify whether the amount of the adipose tissue reduced from a given portion is acceptable by comparing the actual amount of the adipose tissue cavitated with the adipose tissue value calculated using the therapy parameters. If for example, the depth of the adipose tissue ablated exceeds, or is about to exceed a determined value, the feedback module 69 may be configured to raise an alarm, such as a continuous beep, till the user acknowledges receiving the beep (for example by means of the user interface 56). The user may then review the information from the feedback module 69. In this manner, the feedback module 69 may avoid any inadvertent errors that could otherwise happen due to human error (user oversight).

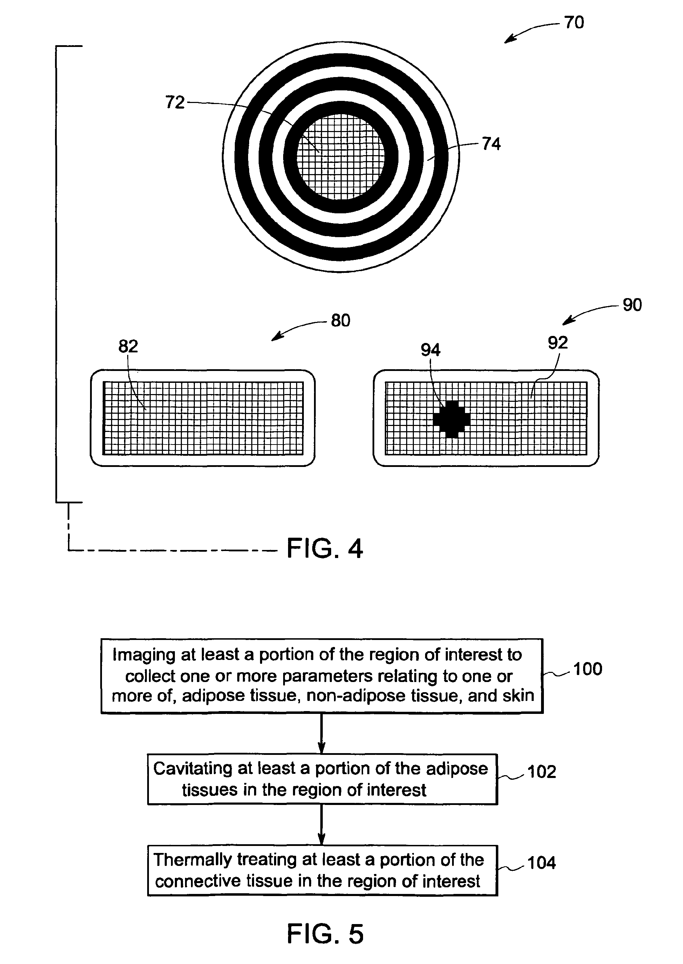

Figure 4 illustrates transducers 70, 80, and 90 that may be used with a probe (not shown) in accordance with various embodiments. The transducers 70, 80, and 90 may include reconfigurable arrays. Typically, the therapy module 54 (FIG. 3) may control the probe 64 (FIG. 3) to deliver low energy imaging pulses and high-energy therapy pulses, respectively. More specifically, the transducer 70 has an imaging array 72 and a separate therapy array 74 that surrounds the imaging array 72. The imaging pulses and the therapy pulses may be delivered separately or in an overlapping manner. The transducer 80 includes an array 82 where the entire array may be used for both imaging and therapy. However, the transducer 90 has an array 92 of transducer elements where a therapy portion 94 of the array 92 may be activated to provide therapy. For example, the controller (FIG. 3) may drive a subset (e.g., the therapy portion 94) of the transducer elements of the array 92 based on the user inputs designating the treatment space.

FIG. 5 is a flow chart for a non-invasive method for substantially contemporaneously cavitating and thermally treating a region of interest.

At block 100, a region of interest is imaged to collect one or more parameters relating to adipose tissue and/or non-adipose tissue (such as connective tissue). The imaging may be done either before and/or after delivering the therapy.

The imaging may be completed before delivering the therapy, for example, to collect the parameters such as but not limited to, anatomical parameters, such as the location, shape, thickness, and orientation of adipose tissue and non-adipose tissues. The delivery of the therapy may be planned according to the parameters determined using imaging, and any other factors already known to the user, or provided by the patient. An anatomical parameter may also include a density of the adipose tissue and the non-adipose tissues.

Optionally, before delivering the therapy, the user may use an imaging instrument such as a diagnostic ultrasound device, an MRI device, a X-ray device, or a DXA (Dual energy x-ray attenuation) device to determine if there is sufficient adipose tissue depth in a desired area to be treated using HIFU energy. Alternatively, determining a volume of adipose tissue to be treated may include known tests such as a manual pinch test or caliper test carried out by a trained physician to determine if a patient has sufficient adipose tissue at a particular site to warrant the procedure. The safety measure and standard used by such a test can also satisfy the minimum requirements of a HIFU procedure.

Once the volume of tissue is identified, the user may determine the corresponding surface area over the volume that can be treated. The user may create one or more contour lines as part of the treatment-planning phase prior to commencing the therapy. During this step the physician may draw or otherwise indicate on a patient skin surface, a region that can safely be treated using a HIFU transducer. Pens or markers may be used to create these contour lines.

While the depth of the adipose tissue should be sufficient to allow the focal zone of the HIFU transducer to be safely in the adipose tissue with some margin of safety both above and below the focal point of the transducer, it should be understood that varying the focal depth of the transducer, as well as the shape and focus of the transducer can allow for more precise control over the delivery of ultrasound (HIFU) energy, while simultaneously reducing the clearance zones needed for safe operation. That is to say a highly focused transducer may provide sufficient control and focus to allow for a reduced safety clearance.

Once the pre-treatment steps of determining a volume of adipose tissue to be treated, and identifying and/or making a corresponding surface area of skin over the volume of adipose tissue are carried out, an ultrasound probe is moved over the identified region to ablate the underlying adipose tissues, and perform skin tightening.

At block 102, at least a portion of the adipose tissue in the region of interest is cavitated using acoustic energy delivered by the transducer. The acoustic energy for cavitating may have a frequency in a range from about 100KHz to about 2MHz. The selection of frequency value for cavitating the adipose tissue may depend upon power of the transducer, duty cycle of the transducer, and the like. There are a few major factors affecting the frequency selection. For example, the depth of the treatment zone may determine the choice of ultrasound frequency. The choice of a lower frequency to penetrate to deeper tissue may negatively impact the energy deposition, as the lower frequencies are not absorbed at the same rate as higher frequencies. Also, the size of the focus spot may determine the ultrasound frequency. The ultrasound beam with higher frequency may be focused in a smaller region for better spatial resolution. A small focus spot is useful for small treatment areas and thin tissue regions. Small focus region usually means low coverage and slow speed. During a therapy session, the ultrasound frequency may vary from one region of interest to another. For example, a higher frequency may be more appropriate for tissue ablation at small, curvy areas.

The transducer is moved over the surface area identified above. The transducer emits energy to the focal zone in sufficient strength (power) and intensity (pressure) for destroying the adipose tissue. If the transducer is moved in a continuous manner such that a single linear lesion field is formed along the path or axis of motion, the lesion field is said to be contiguous, or a contiguous lesion field. A volume of over lapping lesion field produced from more than one scan line (such as an intersection) forms a cooperative lesion field.

In one embodiment, the HIFU energy may be applied in a manner to form a pattern of discrete ablated field and non-ablated fields around the ablated fields within a region of interest. In another embodiment, the HIFU may be applied in a manner that divides the region of interest into a plurality of smaller treatment sites, and the sum of the treatment sites produces the desired coverage to form the region of interest. HIFU energy may be applied in either continuous or discontinuous motion through individual treatment sites. The various treatment sites, which form the region of interest, may be uniform or different in size.

Optionally, at block 103, imaging may be carried out to determine the amount of adipose tissue that is to be cavitated and to determine the effect of therapy in the region of interest. Depending on the amount of the cavitated tissue, a decision may be taken by the user or the system whether to continue cavitating, or to switch to thermally treating the connective tissue. Also, a decision may be made by the user or the system whether the therapy needs to be continued in the same region of interest, or if a new region of interest needs to be selected.

At block 104, the connective tissues of the region of interest, for example, the connective tissue surrounding the cavitated tissues is thermally treated or tightened by acoustic energy having a second frequency range. The frequency range of the thermally treating acoustic energy may be in a range from about 1MHz to about 4MHz so as to be substantially thermal in effect. In one embodiment, the thermal treatment immediately follows the cavitation, however, for example, an imaging step may be performed between the steps of cavitating and thermally treating.

Optionally, thermal treatment may precede adipose tissue cavitation for increasing the effect of the cavitation. Thermally treating the adipose tissues prior to cavitating may increase the efficiency of the cavitating process and decreases the time required for cavitating the adipose tissues.

In certain embodiments, imaging may be carried out after thermally treating the connective tissues to determine whether to continue the treatment of the determined region of interest or move on to the next region of interest in the subject.

Imaging may be carried out in real time, or an image of the region of interest may be created after every treatment step. In one example, the image may be a B-mode, or an elastography image. The user or the feedback module may refer to the images acquired to verify that the treatment delivered to the region of interest is producing desired results or the treatment needs to be modified. For example, the user or the feedback module may refer to the images to confirm if the delivery of the calculated treatment dose produced the desired results, or if more treatment is required to cavitate fat cells in the adipose layer to achieve the desired thickness. Also, the decision to discontinue/stop the therapy to a particular region of interest may be based on the images acquired of the region of interest during or after the therapy.

As mentioned, the steps of imaging, cavitating and thermally treating may be performed by delivering the corresponding acoustic energy to the region of interest. The acoustic energy may be delivered using two or more ultrasound transducers disposed in a single housing. As mentioned, one transducer is used for imaging, whereas the other one or two transducers are used for cavitating and thermally treating. The ultrasound parameters of the transducer may be altered to switch between the step of cavitating and thermally treating. If a single transducer at a single frequency is used for both the cavitational and thermal treatments the transducer switches between the step of cavitating and thermally treating by altering a parameter comprising duty cycle, power, duration of treatment, pulse length, or combinations thereof. The steps of cavitating and thermally treating are provided in the same therapy session, and using the same device.

The combination of cavitating and thermally treating may be applied in several different fashions. In one example, the step of cavitating comprises cavitating the adipose tissue over the entire region of interest, and the step of thermally treating refers to applying thermal treatment subsequent to the step of cavitating and comprises thermally treating the connective tissue over the entire region of interest. In another example, cavitating at a location within the region of interest may be followed by thermally treating the location before shifting the probe to the next location within the region of interest.

In one embodiment, a multi-element ultrasound system is used. When therapy is applied, ultrasonic therapy signals (e.g., HIFU) from the probe are directed toward a region of interest. A treatment location within the region of interest may be defined as a region where a therapy beam formed by ultrasound signals from the transducer elements is focused. In other words, the treatment location includes a focal region of the transducer elements. The therapy beam is shaped and directed by a selected configuration and operation of the transducer elements. As such, the treatment location may vary in size and shape within a single therapy session.

The therapy location throughout the region of interest may be moved between multiple points or locations. As used herein, "moving the treatment location between multiple points" includes, but is not limited to, moving the treatment location along a therapy path between a first point and an end point, and also may comprise moving the treatment location to separate and distinct points within the region of interest that may or may not be adjacent to one another along a path. The therapy path may take the form of separate points where therapy is applied. For example, therapy may first be applied to a first point. After therapy has been applied to the first point, the focal region may be readjusted onto a second point along the therapy path that is separate and remotely spaced from the first point. Therapy may then be applied to the second point. The process may continue along the therapy path until the therapy session is concluded at an end point. In other embodiments, the therapy may be continuously applied as the focal region is moved along the therapy path in a sweeping manner. For example, therapy may be continuously applied as the treatment location is moved between the first point and the end point.

Parameters of the HIFU transducer may be adjusted to produce the desired treatment needed to destroy adipose tissue and denature collagen fibrils. Moving the HIFU transducer and applying therapeutic ultrasound energy generally do not produce lesion or halo fields that extend beyond the dimensions of the adipose tissue volume.

Although the embodiments described above are illustrated as treating adipose tissue, alternative embodiments may be used to treat other tissues within the body. For example, the above-described embodiments may be used to image and treat a tumor within a region of interest. With respect to adipose tissue, various embodiments may be used to automatically identify the tumor and/or to allow user inputs to identify treatment spaces within a region of interest and to set therapy parameters for the treatment. Furthermore, embodiments described herein may be used for palliative treatments for cancer, thermal treatment of muscles, or ultrasonically activating drugs, proteins, stem cells, vaccines, DNA, and gene delivery.

While only certain features of the invention have been illustrated and described herein, many modifications and changes will occur to those skilled in the art. It is, therefore, to be understood that the appended claims are intended to cover all such modifications and changes as fall within the scope of the invention.

## Claims

1. An ultrasound device (20) for cavitating adipose tissue and for treating adipose and connective tissue in a region of interest, the ultrasound device (20) being disposed in a housing (28) and comprising:
a first transducer (22) for imaging the region of interest; and **characterised by**:
a second transducer (24) configured to generate one or more ultrasound frequencies for cavitating fat cells in the region of interest at a cavitating frequency in a range from 100 KHz to 3 MHz, and one or more frequencies for thermally treating connective tissues in the region of interest at a thermal treating frequency in a range from 1 MHz to 30 MHz.

2. The ultrasound device (20) of claim 1, wherein the second transducer (24) is switchable between a pulsed mode and a continuous-wave mode.

3. The ultrasound device (20) of claim 1, wherein the second transducer (24) is configured to generate high intensity focused ultrasound (HIFU).

4. The ultrasound device (20) of claim 1, wherein the first transducer (22) is operable at least in part as a receiver.

## Patentansprüche

1. Ultraschallgerät (20) zur Kavitation von Fettgewebe und zur Behandlung von Fett- und Bindegewebe in einer Region of Interest, wobei das Ultraschallgerät (20) in einem Gehäuse (28) angeordnet ist und Folgendes umfasst:
einen ersten Wandler (22) zur Abbildung der Region of Interest, **gekennzeichnet durch**:
einen zweiten Wandler (24), der konfiguriert ist, um eine oder mehrere Ultraschallfrequenzen zur Kavitation von Fettzellen in der Region of Interest bei einer Kavitationsfrequenz im Bereich von 100 kHz bis 3 MHz, sowie eine oder mehrere Frequenzen zur Wärmebehandlung von Bindegewebe in der Region of Interest bei einer Wärmebehandlungsfrequenz im Bereich von 1 MHz bis 30 MHz zu erzeugen.

2. Ultraschallgerät (20) nach Anspruch 1, wobei der zweite Wandler (24) zwischen Pulsmodus und durchgehendem Wellenmodus umschaltbar ist.

3. Ultraschallgerät (20) nach Anspruch 1, wobei der zweite Wandler (24) so konfiguriert ist, um hochintensiven fokussierten Ultraschall (HIFU) zu erzeugen.

4. Ultraschallgerät (20) nach Anspruch 1, wobei der erste Wandler (22) zumindest teilweise als Empfänger betrieben werden kann.

## Revendications

1. Un dispositif à ultrasons (20) pour la cavitation de tissus adipeux et pour traiter des tissus adipeux et conjonctifs dans une région d'intérêt, le dispositif à ultrasons (20) étant disposé dans un boîtier (28) et comprenant :
un premier transducteur (22) pour acquérir des images de la région d'intérêt ; et **caractérisé par**
un second transducteur (24) configuré pour générer une ou plusieurs fréquences d'ultrasons pour la cavitation de cellules de graisse dans la région d'intérêt à une fréquence de cavitation dans une gamme de 100KHz à 3MHz, et une ou plusieurs fréquences pour traiter thermiquement des tissus conjonctifs dans la région d'intérêt à une fréquence de traitement thermique dans une gamme de 1 MHz à 30MHz.

2. Le dispositif à ultrasons (20) de la revendication 1, dans lequel le second transducteur (24) est commutable entre un mode pulsé et un mode d'onde continue.

3. Le dispositif à ultrasons (20) de la revendication 1, dans lequel le second transducteur (24) est configuré pour générer des ultrasons focalisés de haute intensité (UFHI).

4. Le dispositif à ultrasons (20) de la revendication 1, dans lequel le premier transducteur (22) peut être utilisé au moins en partie comme récepteur.
